# EUROPEAN PATENT SPECIFICATION

(11) **EP 4 262 611 B1**
(45) Date of publication and mention of the grant of the patent: **09.04.2025**
(21) Application number: 22700195.5
(22) Date of filing: 06.01.2022
(51) Int. Cl.: A61M 39/04, A61M 39/02, A61B 5/06, A61B 90/00, A61B 90/98, A61F 2/00, A61F 2/12

(54) **MEDICAL IMPLANT PORT ASSEMBLIES AND RELATED METHODS**
MEDIZINISCHE IMPLANTAT-PORT-ANORDNUNGEN UND ENTSPRECHENDE METHODEN
ASSEMBLAGES DE PORTS D'IMPLANTS MÉDICAUX ET MÉTHODES ASSOCIÉES

(30) Priority: 07.01.2021 US 202163134858 P
(43) Date of publication of application: 25.10.2023
(73) Proprietor: Establishment Labs S.A., 20113 Alajuela (CR)
(72) Inventor: RAMIREZ, Mario, La Garita, Alajuela (CR); DAJLES, Denise, La Garita, Alajuela (CR); CASTRO, Natalia, La Garita, Alajuela (CR); LOO, Gian Franco, La Garita, Alajuela (CR); DE MEZERVILLE, Roberto, La Garita, Alajuela (CR); CHACÓN QUIRÓS, Juan José, La Garita, Alajuela (CR); DELGADO, Juan José, La Garita, Alajuela 20113 (CR)
(74) Representative: Haseltine Lake Kempner LLP
(86) International application number: PCT/IB2022/050090
(87) International publication number: WO 2022/149086

(56) References cited:
- EP-A1- 3 556 317
- WO-A1-2020/028847
- US-A1- 2018 336 381
- US-A1- 2019 247 138

## Description

### TECHNICAL FIELD

The present disclosure relates generally to medical implant components useful for determining the location and/or orientation of the medical implant inside a subject, e.g., a patient. For example, the present disclosure includes port assemblies detectable with an external reader. The port assembly may be configured as an injection port for a medical implant including a tissue expander. Related methods of use of the port assemblies and medical implants are also described.

### BACKGROUND

Implantable medical devices may be implanted into patients for a variety of reasons, including, for example, to improve the clinical condition of a patient or to replace natural patient tissue. Injection ports of tissue expanders provide access to tissue expanders while implanted within a patient. Electromagnetic signals may be used to provide information on the location of an injection port of the tissue expander to facilitate access to the port. When a tissue expander is in an irregular position, such as when a tissue expander has flipped position, the injection port may be inaccessible to a physician or other medical professional, who could inadvertently puncture the tissue expander in an effort to access the injection port. EP 3 556 317 A1 discloses implantable transponders comprising no ferromagnetic parts for use in medical implants; methods for using such transponders, readers for detecting such transponders and methods for using such readers are also described.
US 2019/0247138 A1 discloses a tissue expander comprising a shell defining a chamber, and having a flexible wall and a port disposed through the flexible wall.

### SUMMARY

The present disclosure includes port assemblies useful for providing access to a medical implant and/or determining the location and/or orientation of the implant inside a patient. Included herein are injection ports for tissue expanders comprising features that may provide for increased safety and/or decreased necessity for invasive procedures. While portions of this disclosure refer to tissue expanders and breast implants, the devices and methods disclosed herein may be used with other implantable medical devices, such as, e.g., other implants used in cosmetic and/or reconstruction procedures. Thus, for example, the port assemblies herein may provide distinctive signature(s) via electronic signal(s) and/or image(s) to assist a medical professional to determine the location and orientation of a medical implant in a non-invasive manner. The port assemblies herein may provide for introduction and/or removal of a fluid from the implant (e.g., an injection port of a tissue expander) but are not limited to fluid injection capability.

The claimed invention is defined by independent claims 1 and 14, and includes a tissue expander comprising an integrated port (also referred to herein as a port or port assembly), wherein the integrated port comprises a housing comprising a first portion coupled to a second portion, wherein the first portion and the second portion comprise different materials; and a self-sealing cover (e.g., a dome) over an opening into the housing; wherein the first portion faces the cover and allows transmission of electromagnetic signals through the first portion, the first portion being between the second portion and the cover; and wherein the second portion prevents transmission of electromagnetic signals through the second portion in a direction away from the cover. In some aspects, the integrated port further comprises an electromagnetic coil between the first portion and the second portion of the housing. Each of the first portion of the housing and the cover may comprise a polymer, e.g., the same polymer or different polymers. Additionally or alternatively, the second portion of the housing may comprise a non-ferromagnetic metal or non-ferromagnetic metal alloy. In at least one example, the second portion of the housing comprises aluminum. In some examples, the integrated port does not comprise any ferromagnetic materials.

In at least one example, the housing forms a chamber configured to receive a fluid, the chamber comprising the first portion and a second portion; and the integrated port comprises a coil between the first portion and the second portion. The first portion may be configured as a needle stop. The first portion may allow transmission of electromagnetic signals from the coil while the second portion prevents transmission of electromagnetic signals from the coil. As mentioned above, the first portion may comprise a polymer and/or the second portion may comprise a non-ferromagnetic metal or non-ferromagnetic metal alloy. For example, the second portion may comprise aluminum and/or the first portion may comprise a polymer such as poly-ether-ether-ketone. The cover is self-sealing, e.g., when an injection device such as a needle punctures the cover for introducing or removing fluid from the integrated port. The coil may be contained within a fluid-tight compartment of the housing that prevents contact between the coil and a fluid within the chamber. As mentioned above, in some examples, the integrated port does not comprise any ferromagnetic materials. In at least one example, the housing is cylindrical in shape.

The present disclosure also includes medical implants comprising the integrated ports herein. For example, the implant may comprise a flexible shell, and the cover (e.g., dome) of the integrated port may be coupled to an inner surface or an outer surface of the shell. For example, the cover may be coupled to the shell with an adhesive. In at least one example, the shell of the implant comprises silicone, and the cover of the integrated port comprises silicone or poly-ether-ether-ketone. According to the claimed invention, the medical implant is a tissue expander.

The present disclosure also includes non-invasive methods of locating the integrated ports herein, e.g., providing information about the position, location, and/or orientation of a medical implant that includes an integrated port as disclosed herein. The claimed non-invasive method includes ultrasound imaging and analyzing echoes from the integrated port, and optionally analyzing a presence or absence of RFID signals from the integrated port to determine an orientation of the implant relative to tissue of a patient.

Further disclosed herein is an integrated port comprising a chamber configured to receive a fluid, the chamber comprising a first portion and a second portion, wherein the first portion comprises a material different from a material of the second portion, one of the materials being a non-ferromagnetic metal; a coil between the first portion and the second portion; and a cover (e.g., a dome) covering an opening into the chamber; wherein the first portion faces the dome, the first portion being configured as a needle stop; and wherein the first portion allows transmission of electromagnetic signals from the coil while the second portion prevents transmission of electromagnetic signals from the coil. In some examples, the integrated port further comprises an integrated circuit chip coupled to the coil, for example soldered to the coil. Medical implants comprising such integrated ports may comprise a flexible shell, e.g., the cover of the integrated port being coupled to an inner surface or an outer surface of the shell.

### BRIEF DESCRIPTION OF THE DRAWINGS

The accompanying drawings, which are incorporated in and constitute a part of this application, illustrate various examples and together with the description, serve to explain the principles of the present disclosure. Any features of an embodiment or example described herein (e.g., device, method, etc.) may be combined with any other embodiment or example, and are encompassed by the present disclosure.
FIGS. 1A-1C show an exemplary integrated port, according to some aspects of the present disclosure.
FIGS. 2A and 2B show portions of an exemplary integrated port, according to some aspects of the present disclosure.
FIG. 3 shows an exemplary detector device, according to aspects of the present disclosure.
FIGS. 4A and 4B show portions of an exemplary integrated port, according to some aspects of the present disclosure.
FIG. 5 shows an exemplary port coupled to a medical implant, according to some aspects of the present disclosure.
FIGS. 6A-6D illustrate another exemplary port, according to some aspects of the present disclosure.

### DETAILED DESCRIPTION

Aspects of the present disclosure are described in greater detail below. The terms and definitions as used and clarified herein are intended to represent the meaning within the present disclosure. The terms and definitions provided herein control, if in conflict with terms and/or definitions incorporated by reference.

The singular forms "a," "an," and "the" include plural reference unless the context dictates otherwise. The terms "approximately" and "about" refer to being nearly the same as a referenced number or value. As used herein, the terms "approximately" and "about" generally should be understood to encompass ± 5% of a specified amount or value.

As used herein, the term "posterior" refers to the back of a patient, and the term "anterior" refers to front of a patient. Thus, the posterior side of an implant such as a tissue expander or breast implant is the side of the implant facing the chest wall, while the anterior side is the opposite side closest to the skin.

The present disclosure generally relates to medical implants, features of medical implants, port assemblies useful for medical implants, and transponders and sensors for use with such implants and port assemblies, and methods of using such port assemblies, transponders, sensors, and implants. Aspects of the present disclosure may be useful for locating medical devices that may be implanted in the patient. Such implantable medical devices may include, but are not limited to, tissue expanders, breast implants, gluteal implants, and other medical devices in the field of aesthetic or reconstructive surgery, as well as other types of medical devices configured for temporary or permanent implantation inside a patient. Various aspects of the present disclosure may be used with and/or include one or more features of transponders, valve assemblies, integrated ports, and readers disclosed in U.S. Patent No. 10,176,412.

In the invention, the medical implant is a tissue expander. Tissue expanders of the present disclosure may be inflated manually and/or electronically, e.g., with a syringe or other suitable device for introducing and withdrawing a fluid (e.g., a liquid or gaseous fluid) or gel into the tissue expanders. Tissue expanders and other medical implants according to the present disclosure may comprise a port having components useful for determining the location and/or orientation of the tissue expander or other medical implant in a patient, e.g., an integrated port. The terms "integrated port" and "port assembly" are used interchangeably herein to describe ports with features that facilitate identifying the location, position, and/or orientation of a medical implant that includes the integrated port or port assembly. The integrated port may include features to facilitate locating and identifying the port, including in cases when the tissue expander or other medical device is misplaced or suspected of being misplaced, in a non-invasive manner.

For example, the integrated port may comprise an electromagnetic coil configured to emit electromagnetic signals, e.g., radio-frequency identification (RFID) signals, and/or ultrasonic signals. Optionally, the coil may be coupled to an integrated circuit chip and/or other electronic components, including sensors configured to assure medical device integrity such as rupture detection and/or measure patient vitals and/or parameters such as temperature, pressure, heart rate, respiratory rate, and/or pH, among others.

The integrated ports herein allow for non-invasive methods of detecting if a tissue expander implanted in a patient has been misplaced (e.g., flipped such that the port is in a posterior position towards the patient's chest rather than an anterior position towards the skin surface) and thus whether an additional medical procedure should be performed to reposition the tissue expander. For example, the methods herein may prevent or reduce the risk of inadvertently puncturing a tissue expander, creating a deflation and/or resulting in other complications for the patient. The present disclosure also includes methods of locating the port of a tissue expander via ultrasound imaging, including locating the port independent of RFID location.

One or more portions of the integrated port (or port assembly) may comprise one or more non-ferromagnetic materials. Exemplary non-ferromagnetic materials include, but are not limited to, polymers such as poly-ether-ether-ketone (PEEK), polycarbonate, and other plastics; ceramic; silica (e.g., glass); and non-ferromagnetic metals such as aluminum and copper, and alloys of these metals, as well as other metals and metal alloys that are non-ferromagnetic. In some examples, the integrated port may comprise nickel. For example, the integrated port (or port assembly) may comprise one or more materials, all of which may be non-ferromagnetic, to maintain magnetic resonance imaging (MRI) compatibility. The structure of the integrated port and combination of materials may provide for distinctive signals and/or images to assist a medical professional in determining the location, position, and/or orientation of the medical implant via an external reader and/or imaging.

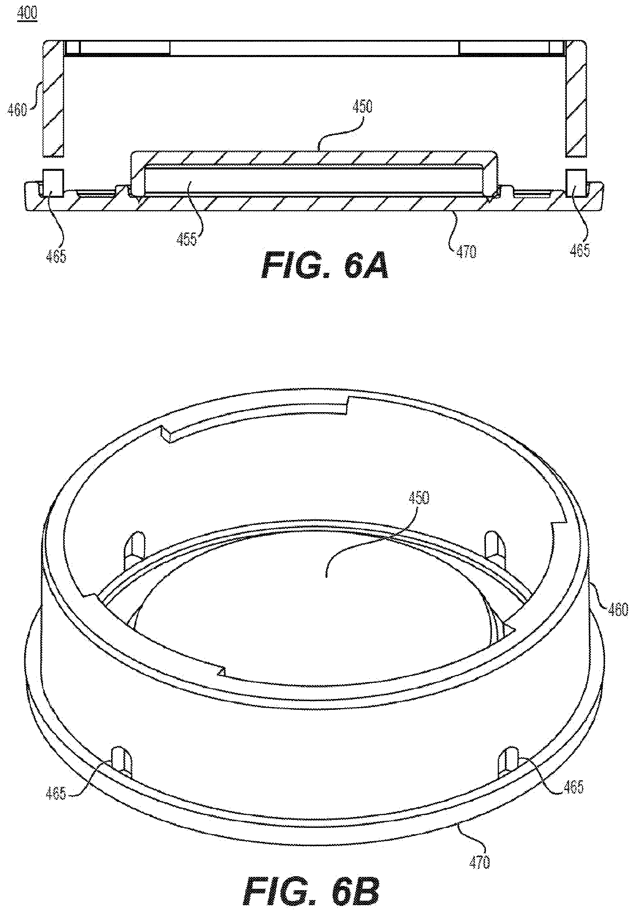

The integrated port comprises at least two different materials. For example, the integrated port may comprise two different metals or metal alloys, a polymer and a metal or metal alloy, or two different polymers, among other combinations of materials. In at least one example, the integrated port comprises a polymer, such as PEEK, and an additional material that has capabilities of RFID blocking, such as aluminum. See, e.g., FIGS. 6A and 6B discussed below. The RFID-blocking material may be positioned so as to block an RFID signal of the integrated port in at least one direction.

In the case of an injection port for a tissue expander, for example, the integrated port may include an electromagnetic coil proximate a needle stop. In at least one example, the RFID-blocking material may be placed adjacent to or on the bottom of a needle stop of the integrated port, or as a "coin" within the needle stop. The coil that emits an RFID signal to assist in locating the integrated port may be proximate, e.g., sit on top of, this RFID-blocking material. Accordingly, the RFID signal may only be detected by a suitable reader/port locator if the RFID-blocking material is not positioned between the coil and the reader/port locator. If the tissue expander has flipped such that the RFID blocking material is between the coil and the reader/port locator, signals emitted from the integrated port may be blocked from detection by the reader/port locator. Failure to detect the integrated port via the port locator may turn on an indicator light of the port locator, e.g., to indicate that the tissue expander is incorrectly positioned, such as due to having flipped or otherwise becoming misplaced.

According to the claimed invention, port location is performed via ultrasound imaging. For example, a difference in materials included in the integrated port allows for the ultrasound echo to differ. The materials may have different densities, chemical compositions, and/or structure, for example, that provide different signatures via ultrasound imaging. This difference in materials allows a medical professional to analyze ultrasound images of a patient to determine the location, position, and/or orientation of a medical implant in a non-invasive manner.

Ultrasound, also referred to as sonography, is a non-invasive medical imaging technique useful for observing the environment surrounding a medical implant. Ultrasound can be used to capture internal images of the body, such as muscles, organs, blood vessels, and other tissue, in addition to implants. An ultrasound machine typically includes a transducer and a processor, such as a central processing unit of a computer, connected to a display, such as a monitor. To collect images, the transducer may be placed on a patient's skin and passed over an area of the body to be imaged, wherein sound waves are emitted from the transducer. When a medical professional obtains images using an ultrasound system, the transducer sends a sound wave with an amplitude that changes due to reflection and transmissions as the sound wave contacts different types of anatomical structure. Reflection and transmission coefficients of the sound waves may be used to describe the environment around an implant. The frequency of the sound waves is typically above 20 kHz, e.g., frequencies ranging between 1 MHz and 10 MHz. The sound waves pass through the body and are at least partially reflected back to the transducer after they bounce off relatively more dense structures, such as bodily tissue or an implant. Echo lines in an ultrasound image are a sign of inhomogeneities in an environment. The processor measures the echo intensities and speed of the sound waves, and converts these measurements to electronic images. The ultrasound images provide an indication of anatomy, e.g., wherein brighter areas correspond to features with greater density. As a sound wave has an initial amplitude that undergoes reflections and transmissions depending on the patient's anatomy and medical implants therein, the reflection and transmission characteristics may be analyzed to calculate describe characteristics of medical implants, including location, position, and/or orientation information.

Ultrasound imaging may be used in addition to, or in place of, electromagnetic detection such as RFID detection. In the case of an integrated port comprising silicone and a polymer (e.g., PEEK), for example, ultrasound may be used to visualize the integrated port independent of RFID detection. This feature may provide detection capabilities for medical implants that do not have an electromagnetic coil. For example, a tissue expander may include an integrated port in the form of an injection port comprising two different materials (e.g., PEEK and silicone, or other combination of different materials) that allow for determining the location, position, and/or orientation of the port via non-invasive ultrasound imaging, independent of locating the integrated port via RFID signal.

In some embodiments, the present disclosure provides an integrated port, comprising: a chamber configured to receive a fluid, the chamber comprising a first portion and a second portion, wherein the first portion comprises a material different from a material of the second portion, one of the materials being a non-ferromagnetic material; a coil between the first portion and the second portion; and a dome covering an opening into the chamber; wherein the first portion faces the dome, the first portion being configured as a needle stop; and wherein the first portion allows transmission of electromagnetic signals from the coil while the second portion prevents transmission of electromagnetic signals from the coil. The integrated port may comprise an integrated circuit chip coupled to the coil, for example soldered to the coil. The integrated port may be part of a medical implant comprising a flexible shell, the cover of the integrated port being coupled to an inner surface or an outer surface of the shell.

Reference is made to the following figures as examples of the present disclosure, but it is understood that the present disclosure is limited to the particular structures and examples illustrated.

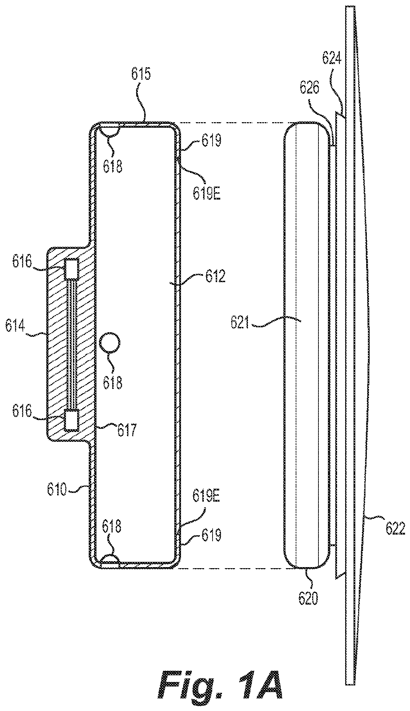

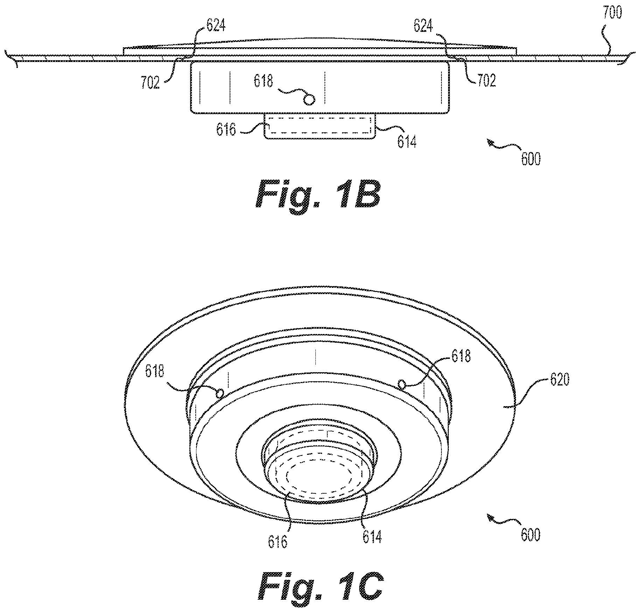

FIGS. 1A-1C depict an exemplary integrated port 600, which may include a valve assembly 610 and a cover in the form of dome 620. Valve assembly 610 may include a main chamber 612 with a wall 615 having a lip 619. Lip 619 has an inner edge 619E. Main chamber 612 may have a top opening defined by edge 619E configured to face dome 620, and may accommodate a portion of dome 620, shown in this example as plug 621 of dome 620. Wall 615 of main chamber 612 may have one or more fluid holes 618 that may pass from main chamber 612 out of the valve assembly 610, e.g., providing for fluid entry and/or exit into a cavity of a medical implant radially inward of the port 600. A coil 616 may be located in a housing 614 which is separated from main chamber 612 by a needle stop 617, such that coil 616 is centered beneath main chamber 612.

Dome 620 may have a patch 622, which may have a wider width than plug 621 and valve assembly 610, and may be integral with plug 621. A flange 626 between patch 622 and plug 621 may be configured to accommodate and interlock with lip 619 of valve assembly 610. In this example, dome 620 is illustrated with a step 624 that may be configured to interface with a wall of an implant (e.g., the shell of a tissue expander, breast implant, or other medical implant) into which the integrated port 600 may be installed, e.g., the dome 620 being located radially outside the implant wall and the main chamber 612 being radially inside the implant wall. This is illustrated in FIG. 1B wherein wall 700 on a medical implant has an aperture 702 through which the port 600 (e.g., plug 621 of port 600) extends.

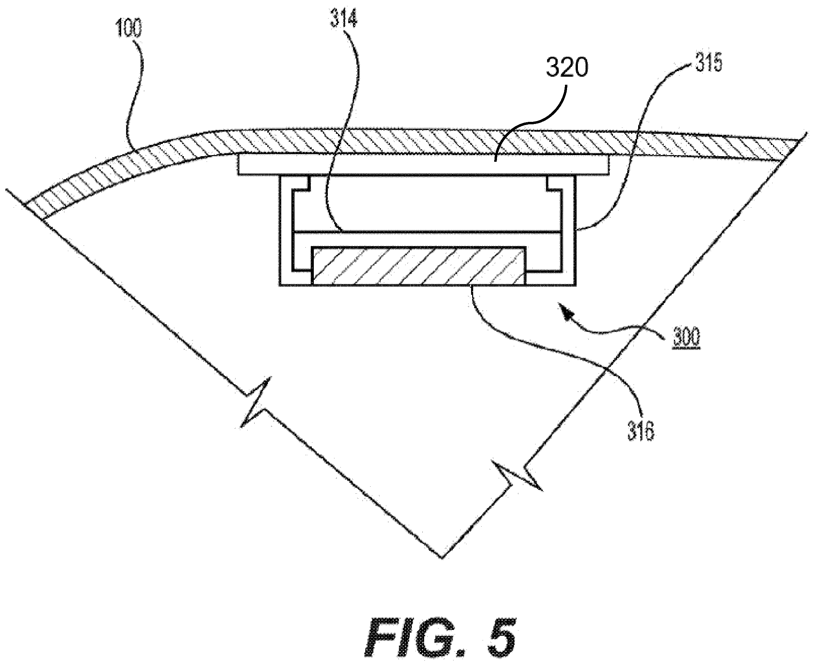

In some examples, the dome 620 may be coupled to the inner surface of a wall of an implant in use, e.g., via an adhesive or other fixation mechanism or material. In such examples, the entire port 600 would be radially inward of the implant wall (e.g., the shell of a tissue expander, breast implant, or other medical implant). In such cases, the port 600 may lack a step 624. For example, the step 624 in FIG. 1A may be omitted, such that the flange 626 is between dome 620 and plug 621. In this example, the wall 700 of implant shown in FIG. 1B would be adjacent to the dome 620 such that the entirety of the port 600 is radially inward of wall 700. See also FIG. 5 discussed below.

Valve assembly 610 may comprise biocompatible, non-ferromagnetic material(s), such as PEEK, silicone, and/or other polymer(s), and/or one or more RFID blocking materials such as aluminum, nickel, copper, and/or alloys thereof. Main chamber 612 may be sized, shaped, and configured to receive fluids from, e.g., a cannula, syringe, or other fluid deposition device. Needle stop 617 of main chamber 612 may be configured to prevent or resist puncturing by such a fluid deposition device (e.g., a needle of a syringe). For example, needle stop 617 may comprise one or more materials having a density, hardness, and/or thickness configured to prevent or resist puncturing by a needle. According to some aspects of the present disclosure, integrated port 600 may include a needle stop 617 that comprises a polymer such as PEEK and an RFID-blocking material such as aluminum opposite the needle stop 617.

Coil 616 may be an electromagnetic coil positioned within a separate chamber, e.g., provided in this example by housing 614. Housing 614 may be fluid-tight so as to prevent fluids from entering housing 614 and coming into contact with coil 616. Housing may be cylindrical, as shown, and may be coaxial with main chamber 612, such that coil 616 is also coaxial with main chamber 612. In this manner, the location of coil 616 may be used to locate the center, or the approximate center, of main chamber 612 and housing 614. Housing 614 is depicted as having a smaller circumference than, e.g. main chamber 612. However, in some examples, housing 614 may have dimensions as large or nearly as large as main chamber 612. Coil 616 optionally may be coupled to a chip, e.g., an integrated circuit chip as mentioned above. For example, the chip may be soldered or otherwise in electronic contact with the inner or outer surface of coil 616. Plug 621 of dome 620 may be sized and shaped to snugly interlock with, e.g., lip 619 of main chamber 612. Dome 620 may comprise a biocompatible material such as silicone with self-sealing capabilities.

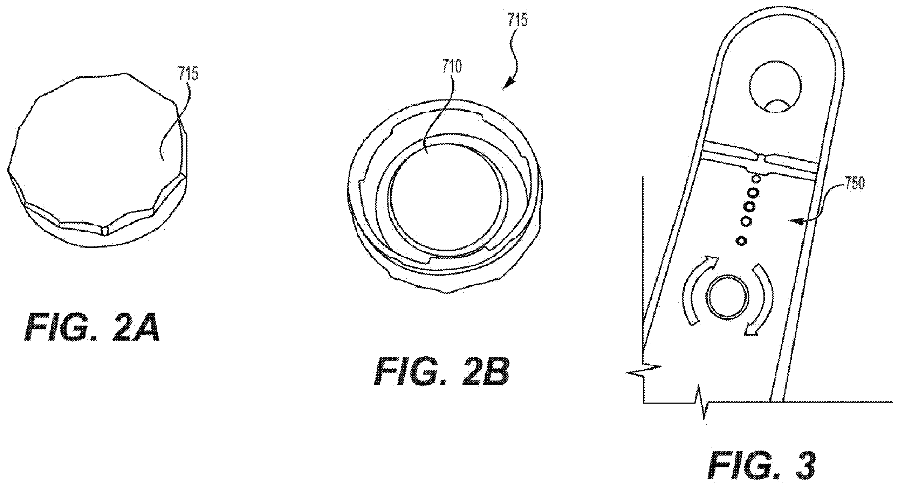

FIGS. 2A and 2B illustrate back and front views, respectively, of components of another exemplary port assembly. In this example is shown a needle stop of the integrated port, which may also include any of the features of integrated port 600 discussed above. The components may include a first portion 710 configured to serve as a needle stop (e.g., similar to needle stop 617) and a second portion 715 coupled to the opposite side of the first portion 710 serving as the needle stop. The first portion 710 may comprise one or more materials configured to prevent or resist puncturing. The first portion 710 may overlay an electromagnetic coil (e.g., similar to coil 616) capable of emitting a RFID signal. The material(s) of first portion 710 may be non-ferromagnetic and may permit the transmission of RFID signals therethrough. Exemplary materials useful for first portion 710 may include, for example, polymers such as PEEK.

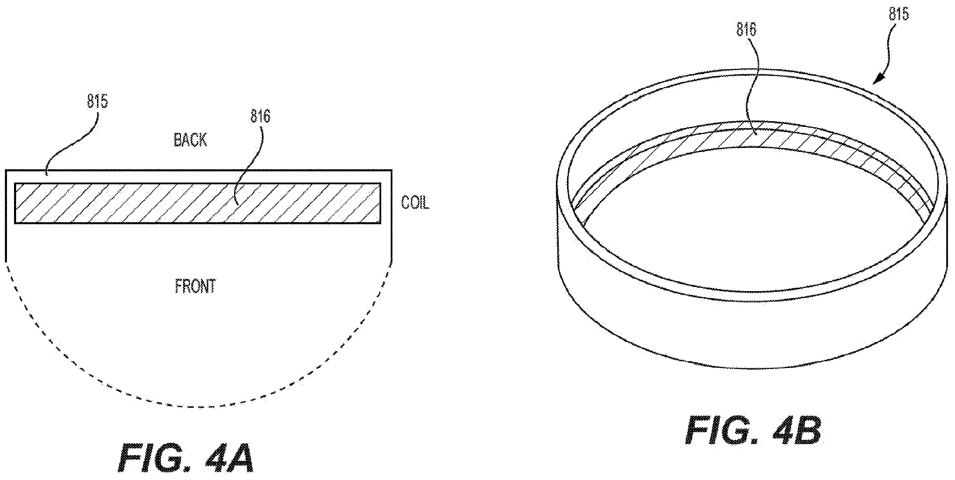

Second portion 715 may comprise a RFID blocking material, such as aluminum or other suitable metal or metal alloy (which additionally may be non-ferromagnetic for MRI compatibility). The second portion 715 is positioned so as to block RFID transmission from the coil below the needle stop in the direction opposite first portion 710. For example, in the case of a tissue expander or other medical implant, second portion 715 may face the posterior side of the tissue expander or other medical implant, while the first portion 710 faces the anterior side of the tissue expander or other medical implant. This configuration is further illustrated in the example shown in FIGS. 4A and 4B, wherein a second portion 815 similar to second portion 715 is positioned to block RFID signals from electromagnetic coil 816, e.g., wherein the coil 816 optionally may be between the RFID-blocking material of the second portion 815 and another material that allows the passage of RFID signals therethrough (e.g., a different material of a needle stop or other structure of the port assembly).

FIG. 3 illustrates an exemplary reader or detector 750, also referred to herein as a port locator, that may be used for detecting integrated ports as disclosed herein. In some examples, the detector may include one or more indicators, such as one or more lights, providing information about location, positioning, and/or orientation of the integrated port (and therefore positioning of the tissue expander or other medical implant that includes the integrated port) based on receiving RFID signals from the integrated port or lack of RFID signals received. For example, when RFID signals are not detected (e.g., when RFID-blocking material(s) prevents signals from an electromagnetic coil from being received by the detector 750), one or more of the indicator lights may illuminate to indicate that the tissue expander is misplaced. For example, the tissue expander or other medical implant may have flipped its position such that the anterior side of the implant faces towards the chest of the patient. In this way, detector 750 may enable a user to determine whether the implant has shifted position so as to prevent access to the port. In the case of a tissue expander where the integrated port is an injection port, the user can then avoid inadvertently puncturing the tissue expander when the port is not accessible, and reposition the tissue expander before attempting to introduce and/or remove fluid via the port.

Additionally, as mentioned above, the integrated ports herein may provide for ultrasound imaging for determining whether the medical implant, e.g., tissue expander, breast implant, gluteal implant, etc., is properly placed, e.g., so as to access the integrated port. A difference in materials of the components of the port, such as different materials used for a needle stop component (e.g., first and second portions 710, 715 above) may allow for an ultrasound echo or echoes to determine the location, position, and/or orientation of the integrated port.

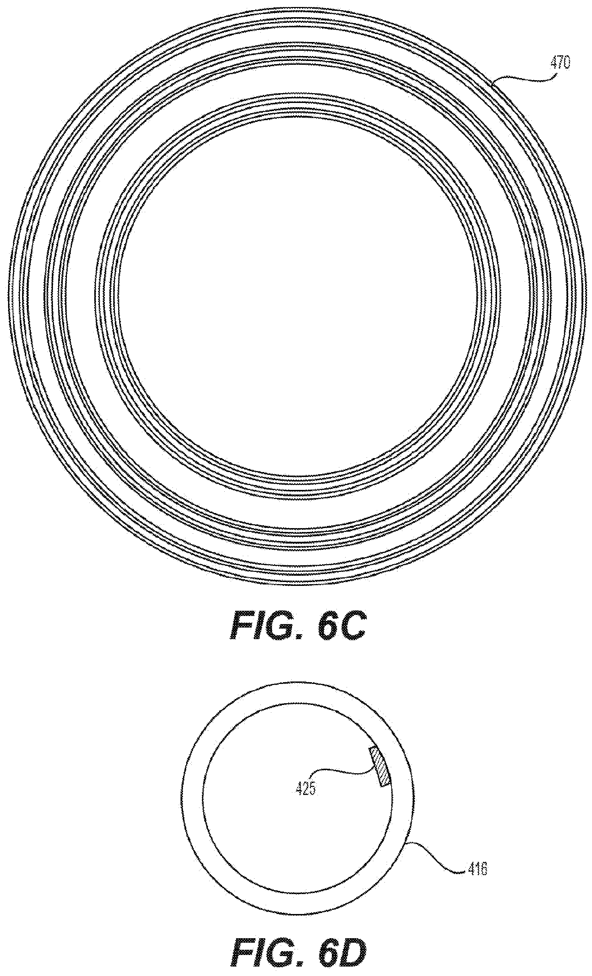

FIG. 5 illustrates another exemplary port assembly 300 (alternatively referred to as integrated port 300) according to some aspects of the present disclosure, the port assembly 300 being coupled to the inner side of the wall 100 of a medical implant. The port assembly 300 includes a cover 320 (or dome) coupled to a lower portion or section that contains an electromagnetic coil 316. As shown, the lower portion or section includes a walled structure 315 coupled to an inner housing 314 that forms a chamber containing the coil 316. Thus, the port assembly 300 comprises a housing that includes the inner housing 314 as a first portion and the walled structure 315 as a second portion, wherein the coil 316 is between the first portion/inner housing 314 and the second portion/walled structure. In some examples, the coil 316 may be coupled to (in electrical contact with) an integrated circuit chip (see, e.g., FIG. 6D discussed below). Optionally, the lower portion or section may include one or more fluid holes to allow the port assembly 600 to serve as an injection port (e.g., the outer surface of the walled structure 315 facing the cover 320 providing a needle stop surface). Thus, port assembly 300 may include any of the features of integrated port 600 of FIGS. 1A-1C. The wall 100 of the medical implant may be a shell (e.g., flexible silicone shell) of a tissue expander or breast implant, for example. When the port assembly 600 is configured to serve as an injection port, a user may introduce and/or remove fluid from the implant via port assembly 600 through a needle inserted through the wall 100 (e.g., through a silicone shell wall of the implant) and through the cover 320 (e.g., the cover comprising a self-sealing material).

According to some aspects of the present disclosure, the inner housing 314 and the walled structure 315 may comprise different materials, one of inner housing 314 or walled structure 315 comprising a material that allows transmission of RFID signals and the other of inner housing 314 or walled structure 315 comprising an RFID-blocking material such as aluminum or other suitable metal or metal alloy, including other non-ferromagnetic metals and metal alloys. Thus, for example, RFID signals emitted by the coil 316 may pass through the inner housing 314 for detection via an external reader or port locator external to a patient, while RFID are blocked from passing through walled structure 315. In this way, a user of the reader or port locator would have information regarding the position, location, and/or orientation of the port assembly 300 to determine if the implant has the proper position, location, and orientation in a patient. A medical professional additionally or alternatively may use ultrasound imaging to determine the position, location, and/or orientation of the port assembly 300, e.g., due to differences in ultrasound echoes of the different materials of inner housing 314 and walled structure 315.

FIGS. 6A-6D illustrate another exemplary port assembly 400, which as shown may serve as an injection port. The port assembly 400 may include components similar to those of port assembly 300 above. For example, the cross-sectional view of FIG. 6A shows a lower portion of the port assembly 400 formed of a walled structure 460, a plate 470, and an inner housing 450 also coupled to the plate 470 to form an inner chamber 455. The walled structure 460 includes two or more holes 465 to allow for the passage of fluid therethrough. The upper surface of the walled structure 460 includes an aperture for receiving a cover, e.g., similar to cover 320 of port assembly 300 shown in FIG. 5 or dome 620 of integrated port 600 shown in FIG. 1A. FIG. 6B shows a perspective view of the port assembly 400 (without cover), illustrating four fluid holes 465 in a cylindrical walled structure 460. The port assembly 600 need not have a cylindrical shape and may have other suitable shapes such as square, rectangular, etc. FIG. 6C shows a top view of the plate 470 which includes several concentric ridges, the innermost ridge configured to abut the inner housing 450 as shown in the cross-sectional view of FIG. 6A. Thus, the port assembly 400 may comprise a housing that includes the inner housing 450 as a first portion, and the walled structure 460 and the plate 470 as a second portion

FIG. 6D shows an exemplary electromagnetic coil 416 that may be housed within the inner chamber 455. The coil is coupled to an integrated circuit chip 425 to provide electrical contact between coil 416 and chip 425. Optionally, chip 425 may store information unique to the port assembly 400 (e.g., a barcode, serial number, identifying information regarding the implant model or manufacturer, etc.) and/or a medical implant that includes the port assembly 400, and/or chip 425 may include one or more sensors for measuring various parameters as discussed above. Coil 416 (and optionally chip 425) may be between the first portion (inner housing 450) and the second portion (walled structure 460 and plate 470) of the housing.

According to some aspects of the present disclosure, the inner housing 450 and the walled structure 460 and/or plate 470 may comprise different materials. For example, one of inner housing 450 or walled structure 460 may comprise a material that allows transmission of RFID signals and the other of inner housing 450 or walled structure 460 may comprise an RFID-blocking material such as aluminum or other suitable metal or metal alloy, including other non-ferromagnetic metals and metal alloys. Additionally or alternatively, one of inner housing 450 or plate 470 may comprise a material that allows transmission of RFID signals and the other of inner housing 450 or plate 470 may comprise an RFID-blocking material such as aluminum or other suitable metal or metal alloy, including other non-ferromagnetic metals and metal alloys. In some examples, the walled structure 460 and the plate 470 may both comprise an RFID blocking material while the inner housing 450 comprises a material that permits transmission of RFID signals therethrough, e.g., a polymer such as PEEK. Thus, for example, RFID signals emitted by the coil 416 (disposed between the inner housing 450 and the plate 470) may pass through the inner housing 450 for detection via an external reader or port locator external to a patient, while RFID are blocked from passing through plate 470 and walled structure 460. A user of the reader or port locator would have information regarding the position, location, and/or orientation of the port assembly 400 to determine if the implant has the proper position, location, and orientation in a patient. A medical professional additionally or alternatively may use ultrasound imaging to determine the position, location, and/or orientation of the port assembly 400, e.g., due to differences in ultrasound echoes of the different materials of inner housing 450, walled structure 460, and/or plate 470.

## Claims

1. A tissue expander comprising an integrated port, wherein the integrated port (300, 400, 600) comprises:
a self-sealing cover (320, 620) over an opening into a housing (314, 614);
**characterized in that**
the housing comprises a first portion (710) coupled to a second portion (715, 815), wherein the first portion and the second portion comprise different materials; and
wherein the first portion faces the cover and allows transmission of electromagnetic signals through the first portion, the first portion being between the second portion and the cover; and
wherein the second portion prevents transmission of electromagnetic signals through the second portion in a direction away from the cover.

2. The tissue expander of claim 1, wherein each of the first portion of the housing and the cover comprises a polymer, optionally wherein the first portion and the cover comprise different polymers.

3. The tissue expander of any one of the preceding claims, wherein the second portion of the housing comprises a non-ferromagnetic metal or non-ferromagnetic metal alloy.

4. The tissue expander of any one of the preceding claims, the integrated port further comprising an electromagnetic coil (316, 416, 616, 816) between the first portion and the second portion of the housing.

5. The tissue expander of claim 4, the integrated port further comprising an integrated circuit chip (425) coupled to the coil, for example soldered to the coil.

6. The tissue expander of any one of claims 4 and 5, wherein:
the housing forms a chamber (612) configured to receive a fluid, the chamber comprising the first portion and the second portion;
the first portion (710) is configured as a needle stop (617, 710); and
the first portion allows transmission of electromagnetic signals from the coil while the second portion prevents transmission of electromagnetic signals from the coil.

7. The tissue expander of any one of claims 4 to 6, wherein the coil is contained within a fluid-tight compartment of the housing (614) that prevents contact between the coil and a fluid within the chamber.

8. The tissue expander of any one of the preceding claims, wherein the first portion comprises a polymer and the second portion comprises a non-ferromagnetic metal or non-ferromagnetic metal alloy.

9. The tissue expander of any one of the preceding claims, wherein the second portion comprises aluminum and/or wherein the first portion comprises poly-ether-ether-ketone.

10. The tissue expander of any one of the preceding claims, wherein the integrated port does not comprise any ferromagnetic materials.

11. The tissue expander of any one of the preceding claims, wherein the housing is cylindrical in shape.

12. The tissue expander of any one of the preceding claims, wherein the implant comprises a flexible shell, and the cover of the integrated port is coupled to an inner surface or an outer surface of the shell, optionally wherein the cover is coupled to the shell with an adhesive.

13. The tissue expander of any one of claims 12, wherein the shell comprises silicone, and the cover of the integrated port comprises silicone or poly-ether-ether-ketone.

14. A non-invasive method of locating the integrated port of the tissue expander of any one of the preceding claims, wherein the method comprises ultrasound imaging and analyzing echoes from the integrated port, optionally wherein the method further comprises analyzing a presence or absence of RFID signals from the integrated port to determine an orientation of the implant relative to tissue of a patient.

## Patentansprüche

1. Gewebeexpander, umfassend einen integrierten Port, wobei der integrierte Port (300, 400, 600) Folgendes umfasst:
eine selbstdichtende Abdeckung (320, 620) über einer Öffnung in ein Gehäuse (314, 614);
**dadurch gekennzeichnet, dass**
das Gehäuse einen ersten an einen zweiten Abschnitt (715, 815) gekoppelten Abschnitt (710) umfasst, wobei der erste Abschnitt und der zweite Abschnitt unterschiedliche Materialien umfassen; und
wobei der erste Abschnitt der Abdeckung zugewandt ist und eine Übertragung von elektromagnetischen Signalen durch den ersten Abschnitt ermöglicht, wobei sich der erste Abschnitt zwischen dem zweiten Abschnitt und der Abdeckung befindet; und
wobei der zweite Abschnitt eine Übertragung von elektromagnetischen Signalen durch den zweiten Abschnitt in einer Richtung weg von der Abdeckung verhindert.

2. Gewebeexpander nach Anspruch 1, wobei jedes des ersten Abschnitts des Gehäuses und der Abdeckung ein Polymer umfasst, wobei der erste Abschnitt und die Abdeckung optional unterschiedliche Polymere umfassen.

3. Gewebeexpander nach einem der vorhergehenden Ansprüche, wobei der zweite Abschnitt des Gehäuses ein nicht ferromagnetisches Metall oder eine nicht ferromagnetische Metalllegierung umfasst.

4. Gewebeexpander nach einem der vorhergehenden Ansprüche, wobei der integrierte Port ferner eine elektromagnetische Spule (316, 416, 616, 816) zwischen dem ersten Abschnitt und dem zweiten Abschnitt des Gehäuses umfasst.

5. Gewebeexpander nach Anspruch 4, wobei der integrierte Port ferner einen integrierten Schaltungschip (425) umfasst, der an die Spule gekoppelt, beispielsweise an die Spule gelötet, ist.

6. Gewebeexpander nach einem der Ansprüche 4 bis 5, wobei:
das Gehäuse eine Kammer (612) bildet, die zum Aufnehmen eines Fluids ausgelegt ist, wobei die Kammer den ersten Abschnitt und den zweiten Abschnitt umfasst;
der erste Abschnitt (710) als Nadelanschlag (617, 710) ausgelegt ist; und
der erste Abschnitt eine Übertragung von elektromagnetischen Signalen von der Spule ermöglicht, während der zweite Abschnitt eine Übertragung von elektromagnetischen Signalen von der Spule verhindert.

7. Gewebeexpander nach einem der Ansprüche 4 bis 6, wobei die Spule in einem fluiddichten Raum des Gehäuses (614) enthalten ist, der einen Kontakt zwischen der Spule und einem Fluid innerhalb der Kammer verhindert.

8. Gewebeexpander nach einem der vorhergehenden Ansprüche, wobei der erste Abschnitt ein Polymer umfasst und der zweite Abschnitt ein nicht ferromagnetisches Metall oder eine nicht ferromagnetische Metalllegierung umfasst.

9. Gewebeexpander nach einem der vorhergehenden Ansprüche, wobei der zweite Abschnitt Aluminium umfasst und/oder wobei der erste Abschnitt Polyetheretherketon umfasst.

10. Gewebeexpander nach einem der vorhergehenden Ansprüche, wobei der integrierte Port keine ferromagnetischen Materialien umfasst.

11. Gewebeexpander nach einem der vorhergehenden Ansprüche, wobei das Gehäuse eine zylindrische Form aufweist.

12. Gewebeexpander nach einem der vorhergehenden Ansprüche, wobei das Implantat eine flexible Schale umfasst und die Abdeckung des integrierten Ports an eine Innenfläche oder eine Außenfläche der Schale gekoppelt ist, wobei die Abdeckung optional mit einem Klebstoff an die Schale gekoppelt ist.

13. Gewebeexpander nach einem der Ansprüche 12, wobei die Schale Silikon umfasst und die Abdeckung des integrierten Ports Silikon oder Polyetheretherketon umfasst.

14. Nichtinvasives Verfahren zum Lokalisieren des integrierten Ports des Gewebeexpanders nach einem der vorhergehenden Ansprüche, wobei das Verfahren Ultraschall-Bildgeben und Analysieren von Echos von dem integrierten Port umfasst, wobei das Verfahren ferner optional Analysieren eines Vorhandenseins oder Nichtvorhandenseins von RFID-Signalen von dem integrierten Port umfasst, um eine Ausrichtung des Implantats relativ zu Gewebe eines Patienten zu bestimmen.

## Revendications

1. Expanseur tissulaire comprenant un port intégré, dans lequel le port intégré (300, 400, 600) comprend :
un couvercle (320, 620) auto-obturant sur une ouverture dans un boîtier (314, 614) ;
**caractérisé en ce que**
le boîtier comprend une première partie (710) accouplée à une seconde partie (715, 815), dans lequel la première partie et la seconde partie comprennent différents matériaux ; et
dans lequel la première partie fait face au couvercle et permet la transmission de signaux électromagnétiques à travers la première partie, la première partie se trouvant entre la seconde partie et le couvercle ; et
dans lequel la seconde partie empêche la transmission de signaux électromagnétiques à travers la seconde partie dans une direction opposée au couvercle.

2. Expanseur tissulaire selon la revendication 1, dans lequel chacune de la première partie du boîtier et du couvercle comprend un polymère, optionnellement, dans lequel la première partie et le couvercle comprennent différents polymères.

3. Expanseur tissulaire selon l'une quelconque des revendications précédentes, dans lequel la seconde partie du boîtier comprend un métal non ferromagnétique ou un alliage métallique non ferromagnétique.

4. Expanseur tissulaire selon l'une quelconque des revendications précédentes, le port intégré comprenant en outre une bobine (316, 416, 616, 816) électromagnétique entre la première partie et la seconde partie du boîtier.

5. Expanseur tissulaire selon la revendication 4, le port intégré comprenant en outre un microcircuit intégré (425) accouplé à la bobine, par exemple soudé à la bobine.

6. Expanseur tissulaire selon l'une quelconque des revendications 4 et 5, dans lequel :
le boîtier forme une chambre (612) configurée pour recevoir un fluide, la chambre comprenant la première partie et la seconde partie ;
la première partie (710) est configurée comme une butée d'aiguille (617, 710) ; et
la première partie permet la transmission de signaux électromagnétiques à partir de la bobine tandis que la seconde partie empêche la transmission de signaux électromagnétiques à partir de la bobine.

7. Expanseur tissulaire selon l'une quelconque des revendications 4 à 6, dans lequel la bobine est contenue à l'intérieur d'un compartiment étanche du boîtier (614) qui empêche le contact entre la bobine et un fluide à l'intérieur de la chambre.

8. Expanseur tissulaire selon l'une quelconque des revendications précédentes, dans lequel la première partie comprend un polymère et la seconde partie comprend un métal non ferromagnétique ou un alliage métallique non ferromagnétique.

9. Expanseur tissulaire selon l'une quelconque des revendications précédentes, dans lequel la seconde partie comprend de l'aluminium et/ou dans lequel la première partie comprend du polyétheréthercétone.

10. Expanseur tissulaire selon l'une quelconque des revendications précédentes, dans lequel le port intégré ne comprend aucun matériau ferromagnétique.

11. Expanseur tissulaire selon l'une quelconque des revendications précédentes, dans lequel le boîtier est de forme cylindrique.

12. Expanseur tissulaire selon l'une quelconque des revendications précédentes, dans lequel l'implant comprend une coque flexible, et le couvercle du port intégré est accouplé à une surface interne ou externe de la coque, optionnellement, dans lequel le couvercle est accouplé à la coque avec un adhésif.

13. Expanseur tissulaire selon l'une quelconque des revendications 12, dans lequel la coque comprend de la silicone et le couvercle du port intégré comprend de la silicone ou du polyétheréthercétone.

14. Procédé non invasif destiné à localiser le port intégré de l'expanseur tissulaire selon l'une quelconque des revendications précédentes, dans lequel le procédé comprend l'imagerie ultrasonore et l'analyse d'échos à partir du port intégré, optionnellement, dans lequel le procédé comprend en outre l'analyse d'une présence ou d'une absence de signaux RFID à partir du port intégré afin de déterminer une orientation de l'implant par rapport à un tissu d'un patient.
